# EUROPEAN PATENT SPECIFICATION

(11) **EP 3 337 598 B1**
(45) Date of publication and mention of the grant of the patent: **03.01.2024**
(21) Application number: 16757599.2
(22) Date of filing: 17.08.2016
(51) Int. Cl.: B01D 61/18, B01D 61/20, B01D 61/22

(54) **RE-CIRCULATION LOOP IN CFF/TFF SINGLE USE FLOW PATH**
RÜCKFÜHRUNGSSCHLEIFE IN EINEM CFF/TFF-STRÖMUNGSWEG ZUM EINMALIGEN GEBRAUCH
BOUCLE DE RECIRCULATION DANS UN TRAJET D'ÉCOULEMENT À USAGE UNIQUE DE FILTRATION À FLUX TRANSVERSAL/TANGENTIEL

(30) Priority: 20.08.2015 IN 2581DE2015; 21.08.2015 IN 2610DE2015
(43) Date of publication of application: 27.06.2018
(73) Proprietor: Cytiva Sweden AB, 751 84 Uppsala (SE)
(72) Inventor: NUTALAPATI, Sasi, Kumar, Bangalore Karnataka 560066 (IN); GEBAUER, Klaus, 751 84 Uppsala (SE); LIDERFELT, Karl, Axel, Jakob, 751 84 Uppsala (SE); KARMARKAR, Nachiket, Bangalore Karnataka 560066 (IN); VERNEKAR, Ajit S., Bangalore Karnataka 560066 (IN); SHARMA, Amit, Kumar, Bangalore Karnataka 560066 (IN); LUNDSTROM, Fredrik, Oskar, 751 84 Uppsala (SE)
(74) Representative: Wu, Ping
(86) International application number: PCT/EP2016/069473
(87) International publication number: WO 2017/029305

(56) References cited:
- EP-A1- 2 907 565
- WO-A1-2011/161609
- US-A1- 2005 023 194
- US-A1- 2006 118 472
- US-A1- 2011 005 984
- US-A1- 2013 029 411

## Description

### BACKGROUND

The present invention relates to an automated crossflow filtration method and system for separating a component of interest from one or more other components in a solution. The invention is of use in the field of purifying biofluids including protein separations, where specific proteins must be separated and purified from cell lysates and cultures.

Cell culture and bioreactors and various biotechnology processes have generated considerable interest in recent years due to increased interest in genetic engineering and biopharmaceuticals. Cells are cultured to make, for example, proteins, receptors, vaccines, and antibodies that are utilize in patient therapy, research, as well as in various diagnostic techniques.

The separation of such molecules is of increasing commercial interest in the chemical, pharmaceutical and biotech industries, including, for example, the production of biological drugs and diagnostic reagents. In particular, the isolation and purification of proteins is of increasing importance because of advances in proteomics (the study of the function of proteins expressed by the human genome). Some proteins may only be present at very low concentrations within a biological sample. As a result, development of isolation and separation techniques is particularly important, especially when large scale production is involved.

In general, when proteins are produced in cell culture, they are either located intracellularly or secreted into the surrounding culture media. Also, the cell lines that are used are living organisms and must be fed with a growth medium, containing sugars, amino acids, growth factors, etc. Separation and purification of a desired protein from such a complex mixture of nutrients as well as cellular by-products, to a level sufficient for characterization, poses a formidable challenge.

Ultrafiltration membranes are characterized by pore sizes which enable them to retain macromolecules. Such macromolecules may have a molecular weight ranging from about 500 to about 1,000,000 daltons. As such ultrafiltration membranes can be used for concentrating proteins. Ultrafiltration is a low-pressure membrane filtration process which can be used to separate solutes up to 0.1 µm in size. As a result, for example, a solute of molecular size significantly greater than that of the solvent molecule can be removed from the solvent by the application of a hydraulic pressure, which forces only the solvent to flow through a suitable membrane (usually one having a pore size in the range of 0.001 to 0.1 µm). As a result, ultrafiltration is capable of removing bacteria and viruses from a solution.

Crossflow filtration ("CFF" also referred to a "tangential flow filtration" (TFF)) systems can be are used in industry applications, such as, for example, manufacturing process separations, waste treatment plants and water purification systems where they can extend the lifetime of filtration membranes by removing and/or preventing the build-up of contaminants and promote consistency of the filtration process with time.

The most commonly used CFF/TFF membrane processes are microfiltration and ultrafiltration. Such processes may be pressure driven and depend upon the "membrane flux", defined as the flow volume over time per unit area of membrane, across the microfiltration or ultrafiltration membrane. At low pressures, the transmembrane flux is proportional to pressure. As a result, by varying the transmembrane pressure difference driving force and average pore diameter, a membrane may serve as a selective barrier by permitting certain components of a mixture to pass through while retaining others. This results in two phases, the permeate and retentate phases, each of which is enriched in one or more of the components of the mixture. The retentate stream is recirculated in the flow circuitry and is pumped across the membrane again in a continuous fashion. Such CFF/TFF systems are used to significantly reduce the volume of the sample solution as a permeate stream is withdrawn from the system. So, the sample solution becomes concentrated when the system is driven in a concentration mode.

CFF/TFF systems have the advantage that due to the direction of the flow of the fluid sample, which is essentially parallel to the membrane surface, an automatic sweeping and cleansing takes place so that higher fluxes and higher throughputs can often be attained with such systems in relation to corresponding normal flow filtration systems. Further, a large fraction of sample flows continuously over the membrane surface so that a clogging and fouling is discouraged in such systems. With respect to these and other advantages, CFF/TFF systems are often used in industrial and/or biotechnological processes.

In an automated CFF/TFF system, buffer and other system treatment solutions need to circulate through the filter and other system components for equilibration prior or subsequent to the separation process. Ideally, such circulation and equilibration of buffer and other system treatment solutions is performed by an automated method without the need for manual intervention.

Some relevant documents are EP2907565, WO2011/161609 and US2005/023194.

### BRIEF DESCRIPTION

Hence, the present invention, as defined by the appended claims, is provided.

### BRIEF DESCRIPTION OF THE DRAWINGS

FIG. 1 illustrates a schematic flow diagram of a CFF/TFF system according to one embodiment thereof.
FIG. 2 illustrates a schematic flow diagram of a CFF/TFF system according to one embodiment thereof that shows a filling operation before the filtration process.
FIG. 3 illustrates a schematic flow diagram of a CFF/TFF system according to one embodiment thereof shows the filtration process.
FIG.4 illustrates a schematic flow diagram of a CFF/TFF system according to one embodiment thereof shows a filling operation of system treatment solution.
FIG. 5 illustrates a schematic flow diagram of a CFF/TFF system according to one embodiment thereof shows a circulation operation of system treatment solution.
FIG. 6 illustrates a schematic flow diagram of a CFF/TFF system according to one embodiment thereof shows a draining operation of system treatment solution.
FIG. 7 illustrates a block diagram of an exemplary computing apparatus.
FIG. 8 illustrates a cabinet for the CFF/TFF system of FIG. 1 (outside view). a) Right side panel, b) Frontal panel, c) Left side panel.
FIG. 9 illustrates the FIG. 8 cabinet, with the panels shown from the inside. Dashed lines indicate electrical connections.

### DETAILED DESCRIPTION

In an automated cross flow filtration system the buffer preferably re-circulates through the filter for equilibration by an automated method without the need for manual intervention. The flow path should be designed for this purpose in a way that can serve the re-circulation loop for recirculating the equilibration buffer for filter conditioning or cleaning solution for filter cleaning and storage. A re-circulation loop in a CFF/TFF single use flow path will address the need for buffer re-circulation in an automated method. This method of automated buffer re-circulation will eliminate manual steps, save time for the filtration process and reduce the volume of buffer or other system treatment solution required. One benefit resulting from these advantages is reduced cost of operating the filtration system. Another benefit resulting from these advantages is that the sterility and/or integrity of the fluid system is not compromised as well as risk for compromising the sterility and/or integrity of the fluid system is greatly reduced by eliminating manual interaction for connecting or disconnecting fluid conduits and containers at the outlet and/or inlet connections of the fluid system during the process.

Biofluids as used herein refers to fluids prepared by biological or pharmaceutical methods and may contain biological agents such as cells, molecules (particularly, valuable proteins), suspended particles, media, buffer, carrier, reaction solution, or other liquid component.

System treatment solutions as used herein refers to buffers including equilibration buffers and other pre-filtration system treatment solutions, post filtration system treatment solutions including washing fluid and/or buffers and storage solutions placed into the system during inactivity.

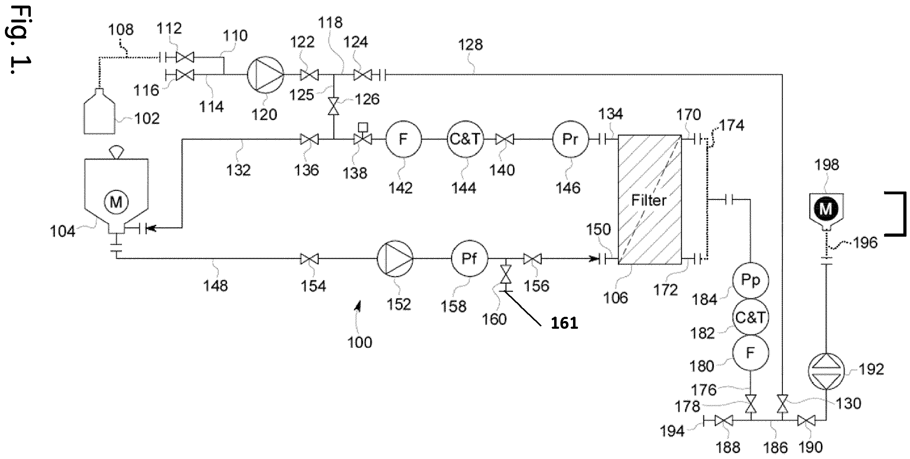

One embodiment of a CFF/TFF system 100 according to the invention, utilizing a microfiltration membrane is shown in FIG. 1, FIG. 8 and FIG. 9. The system may be automated and can be used to separate components present in a solution, such as are commonly found in biological samples including biofluids. For example, depending upon the pore size of the membrane used, cells (such as blood cells) can be washed with buffers prior to lysis to remove contaminants, cellular debris can be separated from soluble materials, and/or proteins can be purified for characterization.

The exemplified embodiment of Fig. 1 includes an automated system 100 that comprises reservoir 102, reservoir 104 and filter 106, for example a CFF/TFF filter or filtration module. The volume of reservoirs 102 and 104 can, for example, range from around 1 liter to around 200 liters in volume and can be scaled to around 2000 liters or more. The reservoirs may be single use or multi-use and can include bags or other vessels composed of suitable material, such as, for example, glass, ceramics or stainless steel and inert plastic material, such as flexible material including flexible plastic material. A means for mixing or agitating the contents of the reservoirs may also be utilized in conjunction with the reservoirs.

The filter 106 may include a microfiltration membrane but it will be understood that, depending upon the nature of the separation to be effected, an ultrafiltration or other membrane could be used. The membrane may be flat or hollow in configuration. A microfiltration membrane may be chosen which has pore sizes such that the component of interest (e.g., a protein) within the solution will pass through the membrane whereas larger components will be retained by it or such that the component of interest (e.g., a protein) within the solution will be retained by the membrane whereas smaller components will pass through it. The material contained therein passing through the membrane is known as the permeate, while the material retained by the membrane is called the retentate.

Suitable membranes may include ultrafiltration, microporous, nanofiltration or reverse osmosis filters formed from polyvinylidene fluoride (PVDF), polysulfone, polyethersulfone, polyarylsulfone, regenerated cellulose, polyamide, polypropylene, polyethylene, polytetrafluoroethylene, cellulose acetate, polyacrylonitrile, vinyl copolymer, polyamides (such as "Nylon 6" or Nylon 66") polycarbonate, PFA, blends thereof or the like.

The system may also includes a plurality of pumps, valves and sensors (sensors as used herein may also includes meters) for driving, regulating, and acquiring data about a solution as it moves through the system. The system may also include an electronic data processing network capable of receiving, transmitting, processing and recording data related to as well as coordinating the operation of the pumps, valves, sensors and the filter. The sensors may be used to gather information and data about the solution passing through the system at various points therein. Such sensors may monitor physical parameters within the system, such as, for example, pressure, temperature, conductivity, pH, oxygen concentration and ultraviolet light absorption as well as volume flow and/or mass flow measurements.

The system includes a plurality of pumps to drive the flow of solution through the system. Although pumps are preferred, other electronically-controllable means for driving a sample solution through such a system may also be used. Pumps used may include, for example, highpressure positive displacement (HPPD) pumps, diaphragm pumps, piston pumps, centrifugal, lobe- and peristaltic pumps as well as other pump configurations including, for example, piezoelectric-driven, acoustically-driven, thermopneumatically-driven and electrostatically-driven pumps. Suitable pump flow rates may range from about 10 ml. per minute to about 1000 liters per minute, preferably about 20 ml. per minute to about 400 liters per minute, depending on feed volume and material to be separated with a stipulated time period.

For certain biopharmaceutical applications in which the biofluid under investigation has substantial and significant protein content, forces and circumstances that can lead to the unintended and undesired denaturation of proteins (i.e., the loss of the physical conformation of the protein's polypeptide constituency) should be avoided and/or mitigated. The mechanical shear forces often produced in the operation of certain pumps, particularly at gas/liquid interfaces (cf. e.g., bubbles), have been linked to protein denaturation, and accordingly, should be mitigated and/or avoided in the selection, manufacture, and incorporation of the device.

Valves are positioned along or otherwise functionally proximate the solution path for regulating the flow of the solution therethrough. The valves are pinch valves that apply a force to flexible tubing passing therethrough to regulate the flow of the solution. The flow of solution through a valve depends upon whether the valve is in an "open" or "closed" state or in some circumstances in an intermediate state, the latter being where the flow of solution is not completely "on" or "off" but in between, thereby regulating the flow rate therethrough.

A conduits system connects reservoir 102, reservoir 104 and filter 106 and so that they are in fluid communication and includes a series of conduits that may also include various valves, pumps and sensors. The filter 106 may include a CFF/TFF filter. There is no particular limitations to the type of conduit used. Potential conduit types include, for example, rigid pipes, flexible tubing, and the channels and passages formed in or intrinsic to the system's other components (e.g., filter, sensors, valves and pumps). Other conduit systems may include "cassettes" that integrate multiple components of the flowpath, for example. For example, tubing networks or fluid conduit networks may be integrated into a single device that may also include valves to regulate the flow through the tubing network, as well as sensors, pumps, filtration modules and other fluid treatment, control or monitoring components. Typically, the plurality of conduits employed in the system may include a mixture of conduit types. Preferably, the majority of the conduits employed are flexible, substantially biologically inert, synthetic polymeric tubing having an internal diameter of 1-50 mm, more preferably 3-32 mm.

In the embodiment of Fig. 1 reservoir 102 is in fluid communication with conduit 108 that is in fluid communication with conduit 110, the latter may include valve 112. A second conduit section includes conduit 114 and valve 116. Conduit 114 may be in fluid communication with another reservoir (not shown) that may be similar to reservoirs 102 and 104. Conduits 110 and 114 are both in fluid communication with conduit 118 that may include pump 120 and valves 122 and 124. Conduit 118 is in fluid communication with conduit 125 that includes valve 126 and with conduit 128 that includes valve 130.

Conduit 125 is in fluid communication with conduit 132 that is connected to retentate outlet 134 of filter 106 and to reservoir 104. Conduit 132 also includes valves 136, 138 and 140 (external tube pinch valves) as well as various sensors including, for example, flow sensor (F) 142, conductivity and temperature sensor (C&T) 144 and pressure retentate sensor (Pr) 146. Conduit (alternatively called a feed conduit) 148 is in fluid communication with reservoir 104 and with conduit 132 via reservoir 104. Conduit 148 is also in fluid communication with feed inlet 150 of filter 106. Conduit 148 may include pump 152, valves 154 and 156 as well as various sensors including, for example, pressure feed sensor (Pf) 158. Valve 160 is also in fluid communication with conduit 148 and a drain/drain line 161 and is connected to a drain/collection/recovery/sample container (not shown).

The embodiment may also include valves and air filters connected thereto such that the valves are in fluid communication with system conduits (not shown). The air filters may be open to the atmosphere or connected to an external integrity tester. Filter 106 also includes permeate outlets 170 and 172 that are both in fluid communication with conduit 174. Conduit 174 is in fluid communication with conduit 176 and further with conduit 186, forming together a permeate conduit. Conduit 176 includes valve 178 as well as various sensors including, for example, flow sensor (F) 180, conductivity and temperature sensor (C&T) 182 and pressure permeate sensor (Pp) 184. Conduits 176 and 128 are in fluid communication with conduit 186 and are thus in fluid communication with each other. Conduit 186 may include valves 188 and 190 and pump 192. Conduit 186 is also in fluid communication with drain 194 and conduit 196. Conduit 196 is in fluid communication with reservoir 198. Reservoirs 104 and 198 may be connected to a means of sensing the mass thereof and as such may be connected to an electronic data processing network capable of receiving, transmitting, processing and recording data therefrom.

A re-circulation loop formed in the flow path includes conduit 128 (recirculation loop conduit) which can connect permeate outlets 170 and 172 via conduits 174, 176 and 186 (the permeate conduit) back to the retentate line/conduit (conduit 132) via conduits 118 and 125. This additional loop is a re-circulation loop that enables a system treatment solution (e.g., equilibration buffer/cleaning or storage solution) to circulate in the flow path until the user drains it via, for example, drain 194. Automated circulation through re-circulation loop minimizes the manual steps involved for filter and system conditioning as well as pre-filtration treatment and post-filtration treatment. Without the re-circulation loop, manual intervention would be needed when using a system treatment solution due to the additional connections/disconnections needed that would involve additional time and threaten user interference in the sterile condition of the system. Thus, utilizing the re-circulation loop, the sterility and/or integrity of the fluid system is not compromised as well as risk for compromising the sterility and/or integrity of the fluid system is greatly reduced by eliminating the manual interaction for connecting or disconnecting fluid conduits and containers at the outlet and/or inlet connections of the fluid system during the process. As a consequence, product (drug) and patient safety are increased. Further, operator safety during production of said products (drugs) is increased when processing potentially harmful fluids, fluid components or product intermediates, which could be the case during vaccine processing or production of ADC (antibody drug conjugates).

Some of the above components may be single use or multi-use depending on the system's usage. If components of the system are to be used more than once, the filter, the membrane therein and other system components can be cleaned using various washing fluid/buffers at the end of a filtration cycle to remove any contaminants (such as solids, particles, etc,) which may, for example, adsorb onto the membrane surface and block the pores. In this way, the operational lifetime of a filter, the membrane therein and other system components can be increased and their efficiency maintained.

The construction materials used in the system can suitably be compatible with commonly used sterilization methods, such as e.g. gamma irradiation and/or autoclaving. For reusable components, stainless steel (e.g. with corrosion resistance at least equivalent to 316L) or engineering plastics such as polysulfone, PEEK, etc. may be used, while for single-use components, plastics, such as, e.g. polysulfone, polypropylene, polyethylene or ethylene copolymers, may be used. Tubing material may comprise materials such as silicone or TPE (thermoplastic elastomers); tubing may be weldable. All materials used in the construction of the system which come into contact with a solution (e.g., system treatment solution), biofluid, retentate and/or permeate are selected to avoid any chemical interaction and to minimize physical adsorption with the components within the solution. Typically, the valves are made of glass, ceramics, stainless steel or external tube pinch valves and the tubing of an inert plastic polymer.

All product contact surfaces of the system and components thereof are desirably, made of FDA compliant and/or USP Class VI tested materials. The system and its components should also be compatible with all commonly used solvents used in CFF/TFF, including, for example, 1N NaOH (at 50° C.), 400 ppm NaOCl (at 50° C.), 1.1% phosphoric acid, 1.8% acetic acid, 2M HCl, 2M urea, "Triton-X" (a non-ionic detergent produced by polymerization of octylphenol with ethylene oxide, available from the Union Carbide Company, Danbury, Conn.), "Tween" (a polysorbate), 30-50% hexalene glycol, 30-50% propylene glycol, 0.07% polysorbate 20, 0.01-0.02% polysorbate 80, 90% ethanol, 90% methanol, 90% isopropyl alcohol, and 25% acetonitrile (w/v water).

To avoid contamination of the biofluid, all system components in contact with the biofluid should be suitably sterilized before the filtration process starts. The system or parts of the system may be assembled and sterilized by autoclaving or radiation, or one or more components may be pre-sterilized and assembled in a sterile system. To facilitate assembly, the system parts or components may be equipped with and connected by suitable connectors, in particular, the sterilized system parts or components may be equipped with and connected by aseptic connectors, e.g. of the ReadyMate type (GE Healthcare). As such, the sterilized system parts or components and associated connectors may be packaged in aseptic packaging. The sterilized system parts/components may also be contained in aseptic packages and assembled in a controlled environment, e.g., a clean room.

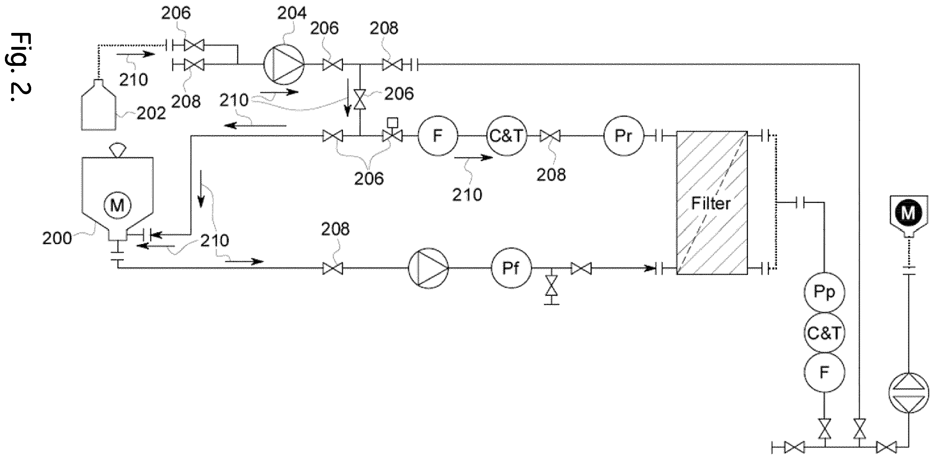

Typical filling operation of an automated CFF/TFF system is exemplified in Fig. 2. Although the solution to be filtered, for example, a biofluid, is contained during operation in reservoir 200, in typical practice, the reservoir 200 may not necessarily be the starting point or origin of the solution. For example, reservoir 200 can be provided with the solution from reservoir 202 through the conduit system shown in Fig. 2 driven by pump 204 with valves 206 in an open position and valves 208 in a closed position with the direction of flow indicated by arrows 210.

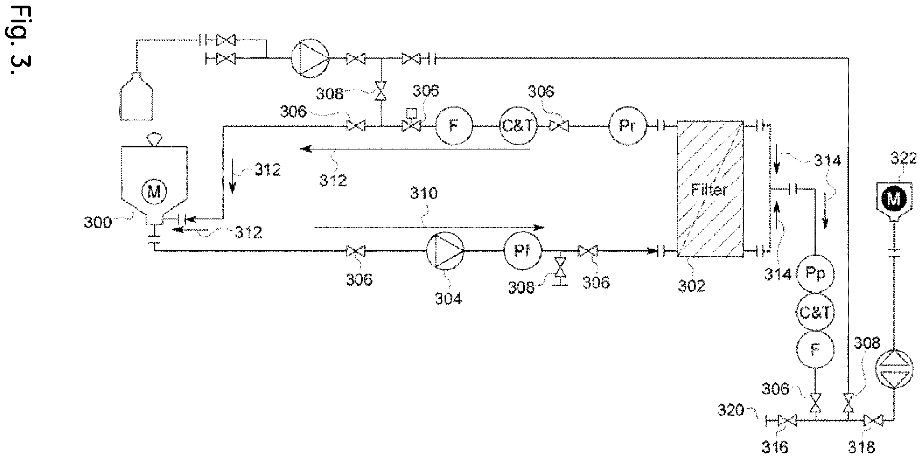

Typical filtration or concentration operation of the CFF/TFF system is exemplified in Fig. 3. The solution to be filtered is contained in reservoir 300 and proceeds through the conduit system shown in Fig. 3 through filter 302 driven by pump 304 with valves 306 in an open position and valves 308 in a closed position with the direction of flow indicated by arrows 310 for the feed, 312 for the retentate and 314 for the permeate. Valve 316 is open and valve 318 is closed when the permeate flow is to be sent to drain 320. Valve 316 is closed and valve 318 is open when the permeate flow is to be sent to reservoir 322. For example, if the solution includes a molecule of interest that is larger than the pore size of the filter membrane, the molecule will be present in the retentate that will be returned to reservoir 300 resulting in a greater concentration of biomolecule present in reservoir 300 as the operation proceeds. If, for example, the solution includes a molecule of interest that is smaller than the pore size of the filter, the molecule will be present in the permeate for collection, for example, in reservoir 322.

Prior to proceeding with the filtration operation, the system may need to treated with equilibration buffer or other pre-filtration treatment solutions to prepare the system for the filtration process. One of the purposes of equilibration buffer and other pretreating solutions may be to prepare the filter and membrane included therein as well as other system components for the fluid containing, for example, a desire biological product (e.g., a protein) by, for example, raising the salt concentration and other fluid physical parameters of the system including the filter, the membrane and other system components to match that of, for example, a bacterial lysate that will be put through the system.

Subsequent to the filtration process, if components of the system are to be used more than once, the filter, the membrane therein and other system components can be cleaned using various washing fluid/buffers at the end of a filtration cycle to remove any contaminants (such as solids, particles, etc,) which may adsorb to the membrane surface and block the pores.

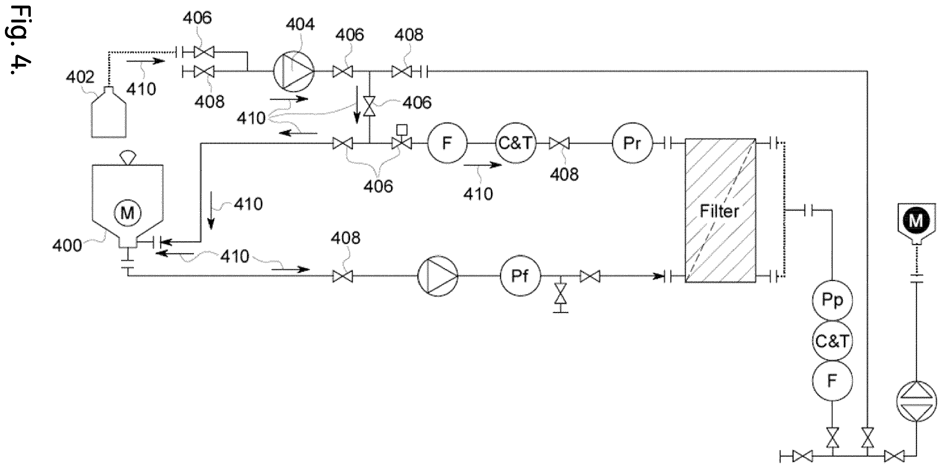

Buffer equilibration and use of other pre-filtration treatment solution as well as use of post filtration washing fluid/buffers and storage solutions (all of which are referred to herein as system treatment solutions) is accomplished by the exemplified embodiment as shown in Figs. 4, 5 and 6. In Fig. 4, the exemplary embodiment undergoes the step of system treatment solution fill in which reservoir 400 may provided with the system treatment solution from reservoir 402 through the conduit system shown in Fig. 4 driven by pump 404 with valves 406 in an open position and valves 408 in a closed position with the direction of flow indicated by arrows 410.

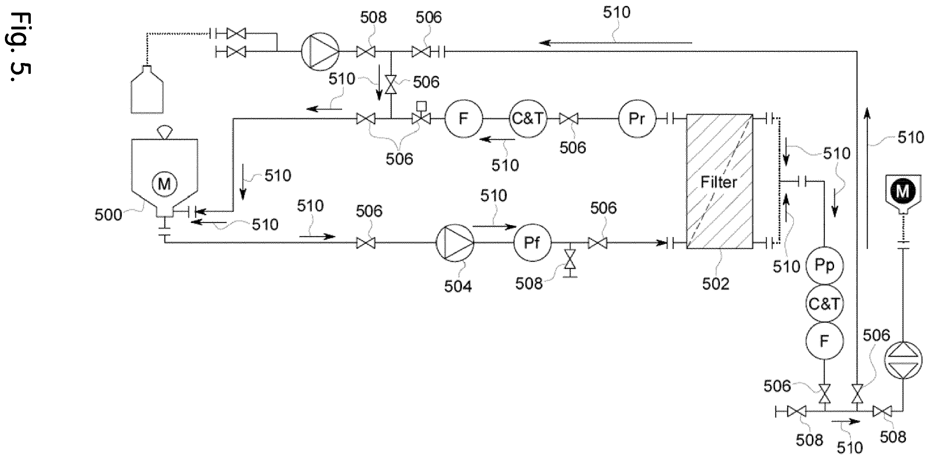

In Fig. 5, the system treatment solution from reservoir 500 is circulated through the conduit system shown in Fig. 5 and filter 502 driven by pump 504 with valves 506 in an open position and valves 508 in a closed position with the direction of flow indicated by arrows 510. Re-circulation is important to the filtration process. For example, buffer needs to recirculate through the filter for filter conditioning at a particular flow for stipulated time and in the same way for the filter cleaning and storage. Conditioning will improve filter efficiency. Suitable pump flow rates during this process may range from about 0.1 liter per hour to about 5000 liters per hour, preferably about 1 liter per hour to about 1000 liters per hour, more preferably 2-300 liters per hour. The conditioning circulation process can take place from about 10 minutes to about 6 hours, however, the time can vary depending on such parameters as, for example, filtration area, filter membrane type, filter condition and process condition.

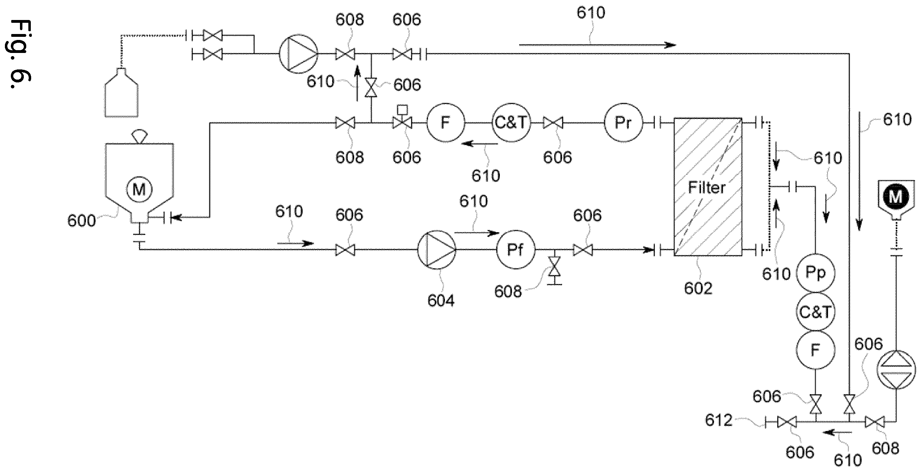

In Fig. 6, after system treatment solution circulation is completed, the system treatment solution is drained from reservoir 600, filter 602 and the conduit system. The draining process is driven by pump 604 with valves 606 in an open position and valves 608 in a closed position with the direction of flow indicated by arrows 610 and to drain 612.

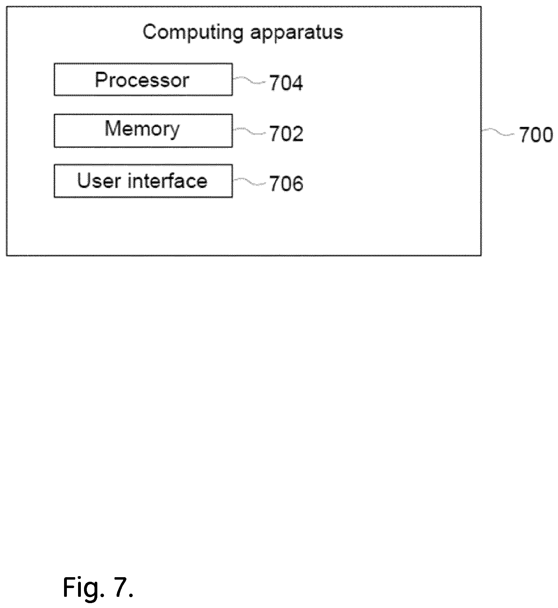

In at least one aspect of the disclosed embodiments, the systems and methods disclosed herein may be executed by an electronic data processing network including, for example, one or more controllers, computers or processor-based components under the control of one or more programs stored on computer readable medium, such as a non-transitory computer readable medium. FIG. 7 shows a block diagram of an exemplary controller or computing apparatus 700 that may be used to practice aspects of the disclosed embodiments. In at least one exemplary aspect, the system control circuitry, data acquisition circuitry, data processing circuitry, operator workstation and other disclosed devices, components and systems may be implemented using an instance or replica of the controller or computing apparatus 700 or may be combined or distributed among any number of instances or replicas of computing apparatus 700.

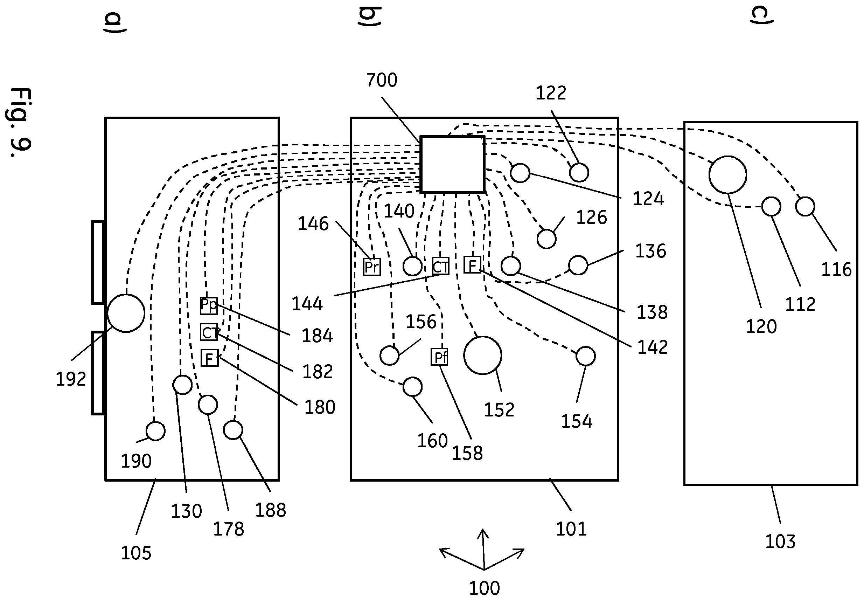

The controller or computing apparatus 700 may include computer readable program code or machine readable executable instructions stored on at least one computer readable medium 702, which when executed, are configured to carry out and execute the processes and methods described herein, including all or part of the embodiments of the present disclosure. The computer readable medium 702 may be a memory of the controller or computing apparatus 700. In alternate aspects, the computer readable program code may be stored in a memory external to, or remote from, the apparatus 700. The memory may include magnetic media, semiconductor media, optical media, or any media which may be readable and executable by a computer. Controller or computing apparatus 700 may also include a processor 704 for executing the computer readable program code stored on the at least one computer readable medium 702. In at least one aspect, computing apparatus may include one or more input or output devices to allow communication among the components of the exemplary imaging system, including, for example, what may be generally referred to as a user interface 706, such as, the operator workstation described above, which may operate the other components included in the imaging system or to provide input or output from the controller or computing apparatus 700 to or from other components of the imaging system. The controller or computing apparatus 700 may be connected to the pumps, valves, sensors and other components of the system (as illustrated in Fig. 9) for coordinating the operation thereof as well as receiving, transmitting, processing, and recording data from the pumps, valves, and sensors.

Preferably, the embodiment of Fig. 1 is a single use system that maximizes maintaining the sterility or integrity of the system during system operation such that the environment outside the system does not contaminate or violate the environment inside the system as well as vice versa. Such operation includes the process of treating the system with system treatment solutions (e.g., buffer, particularly equilibration buffer) and the filtration process itself. In order to maintain the integrity of the system, the embodiment may include a cabinet having sides that define a cabinet interior and cabinet exterior. Some of the components shown in Fig. 1 may be single use and mounted to the cabinet and are at least partially exposed to an external side of the cabinet so as to provide easy access for installation and removal. As a result of utilizing single use components, the system can be set-up and system operation conducted automatically (e.g., utilizing electronic data processing network) without having to change or alter the path of system components that could affect the integrity of the system from the outside or allow components of solutions in the system (e.g., viruses, proteins and other components of a biofluid) to escape the system that could, for example, potentially cause harm to human operators.

Preferably, the complete flow path in fluid contact is provided as a single use flow path unit that is exchangeable in between different processes and production runs. Single-use technology helps reduce time-consuming device preparations, such as cleaning and cleaning validation, and helps reduce a risk for cross-contamination, which is important in applications such as vaccine manufacturing, for example. For example, GE Healthcare Life Science's READYTOPROCESS^{™} platform provides disposable, scalable fluid processing solutions. For example, the ÄKTA ready ^{™} single use chromatography system provides an integrated disposable fluid processing conduit system (Flow Kit) with tubing, sensors, pumps and other fluid treatment, control and monitoring components that comprises all system components in fluid contact aimed for exchange and disposal after processing with biofluids. The Flow Kit is manufactured in a controlled environment and subjected to a bioburden reduction by gamma irradiation.

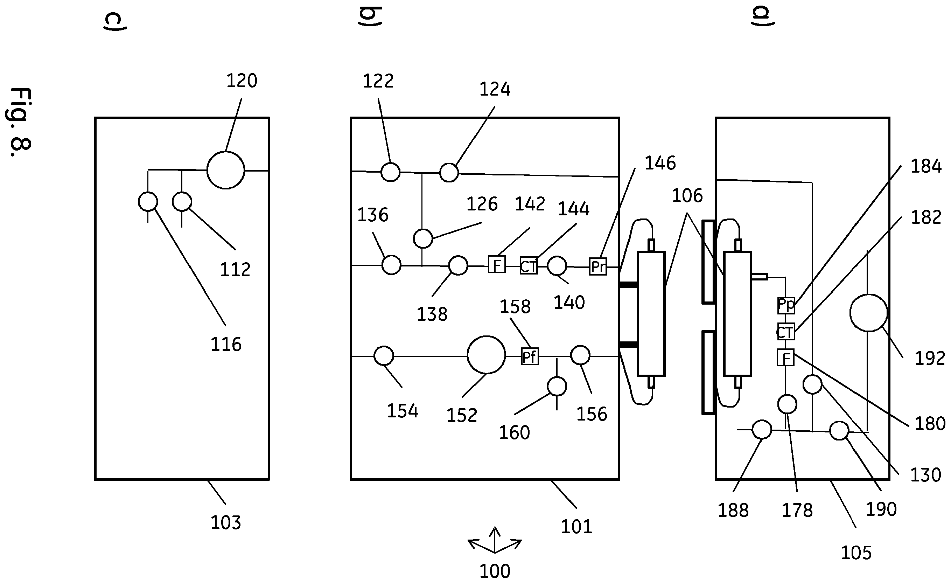

External to the cabinet, for example, in Fig. 1 and the frontal 101, left 103 and right 105 side panels of the cabinet as shown in FIG. 8 and 9 (back side panel not shown) may be reservoirs, conduit tubing, tubing connectors (e.g., aseptic connectors, such as the ReadyMate type (GE Healthcare)), sensors and intersection points to which the conduit tubing encounters other system components, for example pumps and a filter module. For example, some, if not all, of the conduit tubing is flexible tubing, preferably, single use tubing, (e.g., conduits 108, 110, 114, 118, 128, 132, 148, 174, 176, 186 and 196 in Fig. 1) external to the cabinet, including some flexible tubing mounted to an exterior side of the cabinet and other components such as valves, sensors and pumps positioned thereon. The reservoirs, preferably, single use reservoirs, (e.g., reservoirs 102, 104 and 198 in Fig. 1) may be mounted to the cabinet or separate therefrom. The CFF/TFF filter, preferably, a single use CFF/TFF filter, (e.g., filter 106 in Fig. 1) may be mounted to the cabinet such that the portion of the filter connected to the flexible tubing is external to the cabinet, for example, positioned on an external side of the cabinet. The sensors, preferably, single use sensors, (e.g., sensors 142, 144, 146, 158, 180, 182 and 184 in Figs. 1, 8 and 9) may be mounted to the cabinet such that the portion of the sensor connected to the flexible tubing is external to the cabinet, for example, positioned on an external side of the cabinet. The valves (e.g., valves 112, 116, 122, 124, 126, 130, 136, 138, 140, 154, 156, 160, 178, 188 and 190 in Figs. 1, 8 and 9) are pinch valves mounted to the cabinet such that the portion of the valve through which the flexible tubing passes is external to the cabinet, positioned on an external side of the cabinet. The pumps (e.g., pumps 120, 152 and 192 in Figs. 1, 8 and 9) may be peristaltic pumps mounted to the cabinet such that the portion of the peristaltic pump through which the flexible tubing passes is external to the cabinet, for example, positioned on an external side of the cabinet. If another type of pump is utilized (e.g., pump 152 of Fig. 1), for example, a piston pump, the components of the pump that will encounter the solution passing through the system (e.g., inlet, outlet, chamber and parts contained therein, check valve, etc.) may be single use or replaced for each use as well. Furthermore with a piston pump, it may be mounted to the cabinet such that the portion of the piston pump connected to the flexible tubing is external to the cabinet, for example, positioned on an external side of the cabinet. An electronic data processing network including a controller or computing apparatus 700 connected to the pumps, valves, sensors and filter and capable of receiving, transmitting, processing and recording data related to as well as coordinating the operation thereof may be partially or completely internal to the cabinet.

As a result, the operations of system treatment with a system treatment solution and filtration of a solution (e.g., a biofluid) can be accomplished in succession without having to re-configure the system (such as re-routing the fluid conduits) that could compromise the integrity of the system. Furthermore, an electronic data processing network can direct the other components of the system (e.g., pumps and valves as well as sensors and filter) to proceed with the operations of system treatment and filtration of a solution automatically and without human intervention.

This written description uses examples as part of the disclosure, including the preferred mode, and also to enable any person skilled in the art to practice the disclosed implementations, including making and using any devices or systems and performing any incorporated methods.

## Claims

1. An automated CFF system, comprising
a CFF filtration module,
flexible tubing forming a fluid circuitry,
a cabinet having sides (101,103,105) that define a cabinet interior and a cabinet exterior, the cabinet including:
a plurality of pinch valves (112,116,122,124,126,130,136,138,140,154,156,160,178,188,190) such that the portion of the valve through which the flexible tubing passes is positioned on an external side of the cabinet,
a plurality of pumps (120,152,192) such that the portion of the pump through which the flexible tubing passes or is connected is positioned on the cabinet exterior, and
a plurality of sensor connectors including a sensor connected to at least one of the plurality of sensor connectors, the portion of the sensor to which the flexible tubing is connected is positioned on the cabinet exterior;
an electronic data processing network at least partially positioned in the cabinet interior, the electronic data processing network connected to and capable of at least one of receiving, transmitting, processing and recording data associated with at least one of the plurality of pumps, the plurality of pinch valves and the plurality of sensors; wherein
the fluid circuitry includes a first reservoir (104) and a CFF filtration module (106) through which a fluid stream of a solution may be conducted through the automated CFF system, and the fluid circuitry comprises:
a feed conduit (148) to provide fluid communication between an outlet of the reservoir and a feed inlet (150) of the CFF filtration module,
a retentate conduit (132) to provide fluid communication between a retentate outlet (134) of the CFF filtration module and an inlet of the reservoir,
a permeate conduit(174,176,186) to provide fluid communication with a permeate outlet (170,172) of the CFF filtration module, and
a re-circulation loop conduit (128) to provide fluid communication between the permeate conduit and the retentate conduit,
configured such that the system treatment solution, which is an equilibration buffer, a cleaning solution or a storage solution, is automatically circulated from the first reservoir through the feed conduit, the CFF/TFF filtration module, the retentate conduit, the permeate conduit and the recirculation loop conduit to return to the first reservoir.

2. The automated CFF system of claim 1, wherein the plurality of pumps and the plurality of valves are configured to drive and regulate the fluid stream through the automated CFF system.

3. The automated CFF system of claim 1 or 2, wherein the plurality of sensor connectors when connected to a sensor are configured to acquire data about the fluid stream as it flows through the automated CFF system.

4. The automated CFF system of any preceding claim, wherein the electronic data processing network is configured to connect to and determine the performance of the CFF filtration module.

5. The automated CFF system of any preceding claim, wherein the fluid circuitry further includes a second reservoir suitable for containing the solution and having a second reservoir outlet that is in fluid communication with the reservoir.

6. The automated CFF system of any preceding claim, wherein:
a) the plurality of pumps includes a peristaltic pump or a single use piston pump; and/or
b) the flexible tubing, the sensors, the reservoir and the CFF filtration module are single use.

7. The automated CFF system of claim 1, wherein:
a) at least one of the plurality of pumps is a piston pump and the piston pump, the flexible tubing, the plurality of sensors, the reservoir and CFF filtration module are single use; and/or
b) the electronic data processing network is connected to and capable of determining the performance of the CFF filtration module.

8. A method of using an automated CFF system, the method comprising:
providing an automated CFF system (100) configured to utilize flexible tubing comprising:
a cabinet having sides (101,103,105) that define a cabinet interior and a cabinet exterior, the cabinet including:
a plurality of pinch valves (112,116,122,124,126,130,136,138,140,154,156,160,178,188,190) such that the portion of the valve through which the flexible tubing passes is positioned on an external side of the cabinet,
a plurality of pumps (120,152,192) such that the portion of the pump through which the flexible tubing passes or is connected is positioned on the cabinet exterior,
a plurality of sensor connectors such that when a sensor is connected thereto the portion of the sensor through which the flexible tubing is connected is positioned on the cabinet exterior; and
an electronic data processing network at least partially positioned in the cabinet interior, the electronic data processing network connected to and capable of at least one of receiving, transmitting, processing and recording data associated with at least one of the plurality of pumps, the plurality of pinch valves and the plurality of sensors,
wherein the plurality of pumps, the plurality of pinch valves and the plurality of sensors are proximate to a path for flexible tubing configured to form a fluid circuitry including a first reservoir (104), a second reservoir (102), a third reservoir (198) and a CFF filtration module (106) through which a fluid stream of a solution may be conducted through the automated CFF system, the fluid circuitry comprising:
a feed conduit (148) to provide fluid communication between an outlet of the first reservoir and a feed inlet (150) of the CFF filtration module,
a retentate conduit (132) to provide fluid communication between a retentate outlet (134) of the CFF filtration module and an inlet of the first reservoir (104),
a permeate conduit (174,176,186) to provide fluid communication with a permeate outlet (170,172) of the CFF filtration module,
a re-circulation loop conduit (128) to provide fluid communication between the permeate conduit and the retentate conduit,
a second reservoir feed conduit (108,110) to provide fluid communication between the first reservoir and the second reservoir,
a third reservoir feed conduit (196) to provide fluid communication between the first reservoir and the third reservoir, and
a drain (161);
connecting a sensor to at least one of the plurality of sensor connectors;
forming the fluid circuitry using flexible tubing to connect the first reservoir, the second reservoir, the third reservoir, the CFF filtration module, the plurality of pinch valves, the plurality of pumps and the plurality of sensors;
configuring the plurality of pumps and plurality of valves to provide a system treatment solution, which is an equilibration buffer, a cleaning solution or a storage solution, to the first reservoir from the second reservoir;
providing the system treatment solution to the first reservoir from the second reservoir;
configuring the plurality of pumps and plurality of valves to circulate the system treatment solution from the first reservoir through the feed conduit, the CFF filtration module, the retentate conduit, the permeate conduit and the recirculation loop conduit to return to the first reservoir;
circulating the system treatment solution from the first reservoir through the feed conduit, the CFF filtration module, the retentate conduit, the permeate conduit and the recirculation loop conduit to return to the first reservoir;
configuring the plurality of pumps and plurality of valves to drain the system treatment solution from the automated CFF system using the drain;
draining the system treatment solution from the automated CFF system using the drain;
configuring the plurality of pumps and plurality of valves to provide a solution for filtration, which is a biofluid, to the first reservoir from the third reservoir;
providing the solution for filtration to the first reservoir from the third reservoir;
configuring the plurality of pumps and plurality of valves to filter the solution for filtration from the first reservoir through the CFF filtration module; and
filtering the solution for filtration from the first reservoir through the CFF filtration module.

9. The method of claim 8, further including driving and regulating the fluid stream through the automated CFF system using the plurality of pumps and the plurality of valves and optionally further including acquiring data about the fluid stream through the automated CFF system using the plurality of sensors.

10. The method of any one of claims 8-9, wherein the CFF filtration module includes sensors capable of determining the performance thereof, connecting the electronic data processing network to the CFF filtration module so as to at least one of receiving, transmitting, processing and recording data associated with CFF filtration module and determining the performance of the CFF filtration module using the electronic data processing network.

11. The method of any one of claims 8-10, wherein
the drain is in fluid communication with the permeate conduit and the recirculation loop conduit; and
draining the system treatment solution includes circulating the system treatment solution from the first reservoir through the feed conduit, the automated CFF filtration module, the retentate conduit, the permeate conduit, the recirculation loop conduit to the drain such that the flow in the retentate conduit proceeds to the recirculation loop conduit and the flow from the recirculation loop conduit and the flow from the permeate conduit exit the automated CFF system through the drain.

12. The method of any one of claims 8-11, wherein the system treatment solution includes buffer and/or wherein the solution for filtration includes a biofluid.

13. The method of any one of claims 8-12, wherein the electronic data processing network controls the steps of:
configuring the plurality of pumps and plurality of valves to provide a system treatment solution to the first reservoir from the second reservoir;
providing the system treatment solution to the first reservoir from the second reservoir;
configuring the plurality of pumps and plurality of valves to circulate the system treatment solution from the first reservoir through the feed conduit, the CFF/TFF filtration module, the retentate conduit, the permeate conduit and the recirculation loop conduit to return to the first reservoir;
circulating the system treatment solution from the first reservoir through the feed conduit, the CFF filtration module, the retentate conduit, the permeate conduit and the recirculation loop conduit to return to the first reservoir;
configuring the plurality of pumps and plurality of valves to drain the system treatment solution from the automated CFF system using the drain;
draining the system treatment solution from the automated CFF system using the drain;
configuring the plurality of pumps and plurality of valves to provide a solution for filtration to the first reservoir from the third reservoir;
providing the solution for filtration to the first reservoir from the third reservoir;
configuring the plurality of pumps and plurality of valves to filter the solution for filtration from the first reservoir through the CFF filtration module; and
filtering the solution for filtration from the first reservoir through the CFF filtration module,
wherein the fluid circuitry remains unchanged during the above steps.

14. The method of any one of claims 8-13, wherein at least one of the plurality of pumps is a piston pump and the piston pump, the flexible tubing, the plurality of sensors, the first reservoir, the second reservoir, the third reservoir and CFF filtration module are single use.

## Patentansprüche

1. Automatisiertes CFF-System, umfassend
ein CFF-Filtrationsmodul,
flexible Schläuche, die einen Flüssigkeitskreislauf bilden,
einen Schrank, der Seiten (101, 103, 105) aufweist, die ein Schrankinneres und ein Schrankäußeres definieren, wobei der Schrank beinhaltet:
eine Vielzahl von Quetschventilen (112, 116, 122, 124, 126, 130, 136, 138, 140, 154, 156, 160, 178, 188, 190), so dass der Abschnitt des Ventils, durch den der flexible Schlauch verläuft, an einer Außenseite des Schrankes positioniert ist,
eine Vielzahl von Pumpen (120, 152, 192), so dass der Abschnitt der Pumpe, durch den der flexible Schlauch verläuft, oder angeschlossen ist, an der Außenseite des Schrankes positioniert ist, und
eine Vielzahl von Sensoranschlüssen, die einen Sensor beinhaltet, der mit mindestens einem von der Vielzahl von Sensoranschlüssen verbunden ist, wobei der Abschnitt des Sensors, mit dem der flexible Schlauch verbunden ist, am Schrankäußeren positioniert ist;
ein elektronisches Datenverarbeitungsnetzwerk, das zumindest teilweise im Schrankinneren positioniert ist, wobei das elektronische Datenverarbeitungsnetzwerk mit mindestens einer der Vielzahl von Pumpen verbunden ist und zumindest zu einem imstande ist von Daten zu empfangen, zu übertragen, zu verarbeiten und aufzuzeichnen, die mindestens einer der Vielzahl von Pumpen, der Vielzahl von Quetschventilen und der Vielzahl von Sensoren zugeordnet sind; wobei
der Flüssigkeitskreislauf einen ersten Behälter (104) und ein CFF-Filtrationsmodul (106), durch das ein Flüssigkeitsstrom einer Lösung durch das automatisierte CFF-System geleitet werden kann, beinhaltet, und wobei der Flüssigkeitskreislauf umfasst:
eine Zuführleitung (148), um Flüssigkeitskommunikation zwischen einem Auslass des Behälters und einem Zuführeinlass (150) des CFF-Filtrationsmoduls bereitzustellen,
eine Retentatleitung (132), um Flüssigkeitskommunikation zwischen einem Retentatauslass (134) des CFF-Filtrationsmoduls und einem Einlass des Behälters bereitzustellen,
eine Permeatleitung (174, 176, 186), um Flüssigkeitskommunikation mit einem Permeatauslass (170, 172) des CFF-Filtrationsmoduls bereitzustellen, und
eine Rückführschleifenleitung (128), um Flüssigkeitskommunikation zwischen der Permeatleitung und der Retentatleitung bereitzustellen,
so konfiguriert, dass die Systembehandlungslösung, die ein Äquilibrierungspuffer, eine Reinigungslösung oder eine Speicherlösung ist, automatisch vom ersten Behälter durch die Zuführleitung, das CFF/TFF-Filtrationsmodul, die Retentatleitung, die Permeatleitung und die Rückführschleifenleitung zirkuliert, um zum ersten Behälter zurückzukehren.

2. Automatisiertes CFF-System nach Anspruch 1, wobei die Vielzahl von Pumpen und die Vielzahl von Ventilen konfiguriert sind, um den Flüssigkeitsstrom durch das automatisierte CFF-System anzutreiben und zu regulieren.

3. Automatisiertes CFF-System nach Anspruch 1 oder 2, wobei die Vielzahl von Sensoranschlüssen, wenn sie mit einem Sensor verbunden sind, konfiguriert sind, um Daten über den Flüssigkeitsstrom zu erfassen, während dieser durch das automatisierte CFF-System fließt.

4. Automatisiertes CFF-System nach einem vorstehenden Anspruch, wobei das elektronische Datenverarbeitungsnetzwerk konfiguriert ist, um sich mit dem CFF-Filtrationsmodul zu verbinden, und dessen Leistung zu bestimmen.

5. Automatisiertes CFF-System nach einem vorstehenden Anspruch, wobei der Flüssigkeitskreislauf weiter einen zweiten Behälter beinhaltet, der zum Enthalten der Lösung geeignet ist, und einen zweiten Behälterauslass aufweist, der in strömungstechnischer Kommunikation mit dem Behälter steht.

6. Automatisiertes CFF-System nach einem vorstehenden Anspruch, wobei:
a) die Vielzahl von Pumpen eine Schlauchpumpe oder eine Kolbenpumpe zum einmaligen Gebrauch beinhaltet; und/oder
b) der flexible Schlauch, die Sensoren, der Behälter und das CFF-Filtrationsmodul zum einmaligen Gebrauch sind.

7. Automatisiertes CFF-System nach Anspruch 1, wobei:
a) mindestens eine von der Vielzahl von Pumpen eine Kolbenpumpe ist, und die Kolbenpumpe, der flexible Schlauch, die Vielzahl von Sensoren, der Behälter und das CFF-Filtrationsmodul zum einmaligen Gebrauch sind; und/oder
b) das elektronische Datenverarbeitungsnetzwerk mit dem CFF-Filtrationsmodul verbunden ist, und in der Lage ist, dessen Leistung zu bestimmen.

8. Verfahren zur Verwendung eines automatisierten CFF-Systems, wobei das Verfahren umfasst:
Bereitstellen eines automatisierten CFF-Systems (100), das für die Verwendung eines flexiblen Schlauches konfiguriert ist, und umfasst:
einen Schrank, der Seiten (101, 103, 105) aufweist, die ein Schrankinneres und ein Schrankäußeres definieren, wobei der Schrank beinhaltet:
eine Vielzahl von Quetschventilen (112, 116, 122, 124, 126, 130, 136, 138, 140, 154, 156, 160, 178, 188, 190), so dass der Abschnitt des Ventils, durch den der flexible Schlauch verläuft, an einer Außenseite des Schrankes positioniert ist,
eine Vielzahl von Pumpen (120, 152, 192), so dass der Abschnitt der Pumpe, durch den der flexible Schlauch verläuft, oder angeschlossen ist, an der Außenseite des Schrankes positioniert ist,
eine Vielzahl von Sensoranschlüssen, so dass, wenn ein Sensor damit verbunden wird, der Abschnitt des Sensors, durch den der flexible Schlauch verbunden wird, am Schrankäußeren positioniert ist; und
ein elektronisches Datenverarbeitungsnetzwerk, das zumindest teilweise im Schrankinneren positioniert ist, wobei das elektronische Datenverarbeitungsnetzwerk mit mindestens einer der Vielzahl von Pumpen verbunden ist und zumindest zu einem imstande ist von Daten zu empfangen, zu übertragen, zu verarbeiten und aufzuzeichnen, die mindestens einer der Vielzahl von Pumpen, der Vielzahl von Quetschventilen und der Vielzahl von Sensoren zugeordnet sind,
wobei sich die Vielzahl von Pumpen, die Vielzahl von Quetschventilen und die Vielzahl von Sensoren in der Nähe eines Weges für einen flexiblen Schlauch befinden, der konfiguriert ist, um einen Flüssigkeitskreislauf zu bilden, der einen ersten Behälter (104), einen zweiten Behälter (102), einen dritten Behälter (198) und ein CFF-Filtrationsmodul (106) beinhaltet, durch das ein Flüssigkeitsstrom einer Lösung durch das automatisierte CFF-System geleitet werden kann, wobei der Flüssigkeitskreislauf umfasst:
eine Zuführleitung (148), um Flüssigkeitskommunikation zwischen einem Auslass des ersten Behälters und einem Zuführeinlass (150) des CFF-Filtrationsmoduls bereitzustellen,
eine Retentatleitung (132), um Flüssigkeitskommunikation zwischen einem Retentatauslass (134) des CFF-Filtrationsmoduls und einem Einlass des ersten Behälters (104) bereitzustellen,
eine Permeatleitung (174, 176, 186), um Flüssigkeitskommunikation mit einem Permeatauslass (170, 172) des CFF-Filtrationsmoduls bereitzustellen,
eine Rückführschleifenleitung (128), um Flüssigkeitskommunikation zwischen der Permeatleitung und der Retentatleitung bereitzustellen,
eine zweite Behälter-Zuführleitung (108, 110), um Flüssigkeitskommunikation zwischen dem ersten Behälter und dem zweiten Behälter bereitzustellen,
eine dritte Behälter-Zuführleitung (196), um Flüssigkeitskommunikation zwischen dem ersten Behälter und dem dritten Behälter bereitzustellen, und
einen Abfluss (161);
Verbinden eines Sensors mit mindestens einem der Vielzahl von Sensoranschlüssen;
Bilden des Flüssigkeitskreislaufs durch Verwenden eines flexiblen Schlauches zum Verbinden des ersten Behälters, des zweiten Behälters, des dritten Behälters, des CFF-Filtrationsmoduls, der Vielzahl von Quetschventilen, der Vielzahl von Pumpen und der Vielzahl von Sensoren;
Konfigurieren der Vielzahl von Pumpen und der Vielzahl von Ventilen, um eine Systembehandlungslösung, die ein Äquilibrierungspuffer, eine Reinigungslösung oder eine Speicherlösung ist, aus dem zweiten Behälter dem ersten Behälter bereitzustellen;
Bereitstellen der Systembehandlungslösung aus dem zweiten Behälter dem ersten Behälter;
Konfigurieren der Vielzahl von Pumpen und Vielzahl von Ventilen, um die Systembehandlungslösung vom ersten Behälter durch die Zuführleitung, das CFF-Filtrationsmodul, die Retentatleitung, die Permeatleitung und die Rückführschleifenleitung zu zirkulieren, um zum ersten Behälter zurückzukehren;
Zirkulieren der Systembehandlungslösung vom ersten Behälter durch die Zuführleitung, das CFF-Filtrationsmodul, die Retentatleitung, die Permeatleitung und die Rückführschleifenleitung, um zum ersten Behälter zurückzukehren;
Konfigurieren der Vielzahl von Pumpen und Vielzahl von Ventilen, um die Systembehandlungslösung durch Verwenden des Abflusses aus dem automatisierten CFF-System abzulassen;
Ablassen der Systembehandlungslösung aus dem automatisierten CFF-System durch Verwenden des Abflusses;
Konfigurieren der Vielzahl von Pumpen und Vielzahl von Ventilen, um eine Lösung zur Filtration, die eine Bioflüssigkeit ist, vom dritten Behälter dem ersten Behälter bereitzustellen;
Bereitstellen der Lösung zur Filtration aus dem dritten Behälter dem ersten Behälter;
Konfigurieren der Vielzahl von Pumpen und Vielzahl von Ventilen, um die zu filtrierende Lösung aus dem ersten Behälter durch das CFF-Filtrationsmodul hindurch zu filtern; und
Filtern der zu filtrierenden Lösung aus dem ersten Behälter durch das CFF-Filtrationsmodul hindurch.

9. Verfahren nach Anspruch 8, das weiter das Antreiben und Regulieren des Flüssigkeitsstroms durch das automatisierte CFF-System hindurch durch Verwenden der Vielzahl von Pumpen und der Vielzahl von Ventilen beinhaltet, und optional weiter das Erfassen von Daten über den Flüssigkeitsstrom durch das automatisierte CFF-System hindurch durch Verwenden der Vielzahl von Sensoren beinhaltet.

10. Verfahren nach einem der Ansprüche 8-9, wobei das CFF-Filtrationsmodul Sensoren beinhaltet, die in der Lage sind, dessen Leistung zu bestimmen, das elektronische Datenverarbeitungsnetzwerk mit dem CFF-Filtrationsmodul zu verbinden, um zumindest eines von Empfangen, Senden, Verarbeiten und Aufzeichnen von Daten, die dem CFF-Filtrationsmodul zugeordnet sind, und Bestimmen der Leistung des CFF-Filtrationsmoduls durch Verwenden des elektronischen Datenverarbeitungsnetzwerks durchzuführen.

11. Verfahren nach einem der Ansprüche 8-10, wobei
der Abfluss in strömungstechnischer Kommunikation mit der Permeatleitung und der Rückführschleifenleitung ist; und
das Ablassen der Systembehandlungslösung das Zirkulieren der Systembehandlungslösung aus dem ersten Behälter durch die Zuführleitung, das automatisierte CFF-Filtrationsmodul, die Retentatleitung, die Permeatleitung und die Rückführschleifenleitung zum Abfluss beinhaltet, so dass der Fluss in der Retentatleitung zur Rückführschleifenleitung fortschreitet, und der Fluss aus der Rückführschleifenleitung und der Fluss aus der Permeatleitung das automatisierte CFF-System durch den Abfluss hindurch verlassen.

12. Verfahren nach einem der Ansprüche 8-11, wobei die Systembehandlungslösung Puffer beinhaltet, und/oder wobei die Lösung zur Filtration eine Bioflüssigkeit beinhaltet.

13. Verfahren nach einem der Ansprüche 8-12, wobei das elektronische Datenverarbeitungsnetzwerk die Schritte steuert, zum:
Konfigurieren der Vielzahl von Pumpen und Vielzahl von Ventilen, um dem ersten Behälter eine Systembehandlungslösung aus dem zweiten Behälter bereitzustellen;
Bereitstellen der Systembehandlungslösung aus dem zweiten Behälter dem ersten Behälter;
Konfigurieren der Vielzahl von Pumpen und Vielzahl von Ventilen, um die Systembehandlungslösung aus dem ersten Behälter durch die Zuführleitung, das CFF/TFF-Filtrationsmodul, die Retentatleitung, die Permeatleitung und die Rückführschleifenleitung zu zirkulieren, um zum ersten Behälter zurückzukehren;
Zirkulieren der Systembehandlungslösung vom ersten Behälter durch die Zuführleitung, das CFF-Filtrationsmodul, die Retentatleitung, die Permeatleitung und die Rückführschleifenleitung, um zum ersten Behälter zurückzukehren;
Konfigurieren der Vielzahl von Pumpen und Vielzahl von Ventilen, um die Systembehandlungslösung durch Verwenden des Abflusses aus dem automatisierten CFF-System abzulassen;
Ablassen der Systembehandlungslösung aus dem automatisierten CFF-System durch Verwenden des Abflusses;
Konfigurieren der Vielzahl von Pumpen und Vielzahl von Ventilen, um eine Lösung zur Filtration vom dritten Behälter dem ersten Behälter bereitzustellen;
Bereitstellen der Lösung zur Filtration aus dem dritten Behälter dem ersten Behälter;
Konfigurieren der Vielzahl von Pumpen und Vielzahl von Ventilen, um die zu filtrierende Lösung aus dem ersten Behälter durch das CFF-Filtrationsmodul hindurch zu filtern; und
Filtern der zu filtrierenden Lösung aus dem ersten Behälter durch das CFF-Filtrationsmodul hindurch,
wobei der Flüssigkeitskreislauf während der zuvor genannten Schritte unverändert bleibt.

14. Verfahren nach einem der Ansprüche 8-13, wobei mindestens eine von der Vielzahl von Pumpen eine Kolbenpumpe ist, und die Kolbenpumpe, der flexible Schlauch, die Vielzahl von Sensoren, der erste Behälter, der zweite Behälter, der dritte Behälter und das CFF-Filtrationsmodul zum einmaligen Gebrauch sind.

## Revendications

1. Système CFF automatisé, comprenant
un module de filtration CFF,
une tubulure flexible formant un ensemble de circuits de fluide,
une armoire présentant des côtés (101, 103, 105) qui définissent un intérieur d'armoire et un extérieur d'armoire, l'armoire incluant :
une pluralité de vannes à manchon (112, 116, 122, 124, 126, 130, 136, 138, 140, 154, 156, 160, 178, 188, 190) de telle sorte que la partie de la vanne à travers laquelle la tubulure flexible passe, soit positionnée sur un côté externe de l'armoire,
une pluralité de pompes (120, 152, 192) de telle sorte que la partie de la pompe à travers laquelle la tubulure flexible passe ou est raccordée, soit positionnée sur l'extérieur de l'armoire, et
une pluralité de connecteurs de capteur incluant un capteur raccordé à au moins un connecteur de la pluralité de connecteurs de capteur, la partie du capteur à laquelle la tubulure flexible est raccordée, est positionnée sur l'extérieur de l'armoire ;
un réseau de traitement de données électronique positionné au moins partiellement à l'intérieur de l'armoire, le réseau de traitement de données électronique étant raccordé à, et capable de recevoir et/ou de transmettre et/ou de traiter et/ou d'enregistrer des données associées à au moins un élément de la pluralité de pompes, de la pluralité de vannes à manchon et de la pluralité de capteurs ; dans lequel
l'ensemble de circuits de fluide inclut un premier réservoir (104) et un module de filtration CFF (106) à travers lequel un flux de fluide d'une solution peut être acheminé à travers le système CFF automatisé, et l'ensemble de circuits de fluide comprend :
un conduit d'alimentation (148) pour fournir une communication fluidique entre une sortie du réservoir et une entrée d'alimentation (150) du module de filtration CFF,
un conduit de rétentat (132) pour fournir une communication fluidique entre une sortie de rétentat (134) du module de filtration CFF et une entrée du réservoir,
un conduit de perméat (174, 176, 186) pour fournir une communication fluidique avec une sortie de perméat (170, 172) du module de filtration CFF, et
un conduit en boucle de recirculation (128) pour fournir une communication fluidique entre le conduit de perméat et le conduit de rétentat,
configuré de telle sorte que la solution de traitement de système, qui est un tampon d'équilibrage, une solution de nettoyage ou une solution de stockage, soit mise automatiquement en circulation depuis le premier réservoir à travers le conduit d'alimentation, le module de filtration CFF/TFF, le conduit de rétentat, le conduit de perméat et le conduit en boucle de recirculation pour retourner au premier réservoir.

2. Système CFF automatisé selon la revendication 1, dans lequel la pluralité de pompes et la pluralité de vannes sont configurées pour entraîner et réguler le flux de fluide à travers le système CFF automatisé.

3. Système CFF automatisé selon la revendication 1 ou 2, dans lequel la pluralité de connecteurs de capteur, lorsqu'ils sont raccordés à un capteur, sont configurés pour acquérir des données concernant le flux de fluide lorsqu'il s'écoule à travers le système CFF automatisé.

4. Système CFF automatisé selon une quelconque revendication précédente, dans lequel le réseau de traitement de données électronique est configuré pour être raccordé au module de filtration CFF et déterminer les performances de celui-ci.

5. Système CFF automatisé selon une quelconque revendication précédente, dans lequel l'ensemble de circuits de fluide inclut en outre un deuxième réservoir approprié pour contenir la solution et présentant une seconde sortie de réservoir qui est en communication fluidique avec le réservoir.

6. Système CFF automatisé selon une quelconque revendication précédente, dans lequel :
a) la pluralité de pompes inclut une pompe péristaltique ou une pompe à piston à usage unique ; et/ou
b) la tubulure flexible, les capteurs, le réservoir et le module de filtration CFF sont à usage unique.

7. Système CFF automatisé selon la revendication 1, dans lequel :
a) au moins une pompe parmi la pluralité de pompes est une pompe à piston et la pompe à piston, la tubulure flexible, la pluralité de capteurs, le réservoir et le module de filtration CFF sont à usage unique ; et/ou
b) le réseau de traitement de données électronique est raccordé au module de filtration CFF et capable de déterminer les performances du module de filtration CFF.

8. Procédé d'utilisation d'un système CFF automatisé, le procédé comprenant :
la fourniture d'un système CFF automatisé (100) configuré pour utiliser une tubulure flexible comprenant :
une armoire présentant des côtés (101, 103, 105) qui définissent un intérieur d'armoire et un extérieur d'armoire, l'armoire incluant :
une pluralité de vannes à manchon (112, 116, 122, 124, 126, 130, 136, 138, 140, 154, 156, 160, 178, 188, 190) de telle sorte que la partie de la vanne à travers laquelle la tubulure flexible passe, soit positionné sur un côté externe de l'armoire,
une pluralité de pompes (120, 152, 192) de telle sorte que la partie de la pompe à travers laquelle la tubulure flexible passe ou est raccordée, soit positionnée sur l'extérieur de l'armoire,
une pluralité de connecteurs de capteur de telle sorte que, lorsqu'un capteur y est raccordé, la partie du capteur à travers laquelle la tubulure flexible est raccordée, soit positionnée sur l'extérieur de l'armoire ; et
un réseau de traitement de données électronique positionné au moins partiellement à l'intérieur de l'armoire, le réseau de traitement de données électronique étant raccordé à, et pouvant recevoir, transmettre, traiter et/ou enregistrer des données associées à au moins un élément de la pluralité de pompes, de la pluralité de vannes à manchon et de la pluralité de capteurs,
dans lequel la pluralité de pompes, la pluralité de vannes à manchon et la pluralité de capteurs sont à proximité d'un trajet pour une tubulure flexible configurée pour former un ensemble de circuits de fluide incluant un premier réservoir (104), un deuxième réservoir (102), un troisième réservoir (198 ) et un module de filtration CFF (106) à travers lequel un flux de fluide d'une solution peut être acheminé à travers le système CFF automatisé, l'ensemble de circuits de fluide comprenant :
un conduit d'alimentation (148) pour fournir une communication fluidique entre une sortie du premier réservoir et une entrée d'alimentation (150) du module de filtration CFF,
un conduit de rétentat (132) pour fournir une communication fluidique entre une sortie de rétentat (134) du module de filtration CFF et une entrée du premier réservoir (104),
un conduit de perméat (174, 176, 186) pour fournir une communication fluidique avec une sortie de perméat (170, 172) du module de filtration CFF,
un conduit en boucle de recirculation (128) pour fournir une communication fluidique entre le conduit de perméat et le conduit de rétentat,
un deuxième conduit d'alimentation de réservoir (108, 110) pour fournir une communication fluidique entre le premier réservoir et le deuxième réservoir,
un troisième conduit d'alimentation de réservoir (196) pour fournir une communication fluidique entre le premier réservoir et le troisième réservoir, et
un drain (161) ;
le raccordement d'un capteur à au moins un connecteur de capteur de la pluralité de connecteurs de capteur ;
la formation de l'ensemble de circuits de fluide à l'aide d'une tubulure flexible pour raccorder le premier réservoir, le deuxième réservoir, le troisième réservoir, le module de filtration CFF, la pluralité de vannes à manchon, la pluralité de pompes et la pluralité de capteurs ;
la configuration de la pluralité de pompes et de la pluralité de vannes pour fournir une solution de traitement de système, qui est un tampon d'équilibrage, une solution de nettoyage ou une solution de stockage, au premier réservoir à partir du deuxième réservoir ;
la fourniture de la solution de traitement de système au premier réservoir à partir du deuxième réservoir ;
la configuration de la pluralité de pompes et de la pluralité de vannes pour faire circuler la solution de traitement de système depuis le premier réservoir à travers le conduit d'alimentation, le module de filtration CFF, le conduit de rétentat, le conduit de perméat et le conduit en boucle de recirculation pour retourner au premier réservoir ;
la mise en circulation de la solution de traitement de système depuis le premier réservoir à travers le conduit d'alimentation, le module de filtration CFF, le conduit de rétentat, le conduit de perméat et le conduit en boucle de recirculation pour retourner au premier réservoir ;
la configuration de la pluralité de pompes et de la pluralité de vannes pour drainer la solution de traitement de système à partir du système CFF automatisé à l'aide du drain ;
le drainage de la solution de traitement de système à partir du système CFF automatisé à l'aide du drain ;
la configuration de la pluralité de pompes et de la pluralité de vannes pour fournir une solution pour une filtration, qui est un biofluide, au premier réservoir à partir du troisième réservoir ;
la fourniture de la solution pour une filtration au premier réservoir à partir du troisième réservoir ;
la configuration de la pluralité de pompes et de la pluralité de vannes pour filtrer la solution pour une filtration depuis le premier réservoir à travers le module de filtration CFF ; et
la filtration de la solution pou une filtration depuis le premier réservoir à travers le module de filtration CFF.

9. Procédé selon la revendication 8, incluant en outre l'entraînement et la régulation du flux de fluide à travers le système CFF automatisé à l'aide de la pluralité de pompes et de la pluralité de vannes et, facultativement, incluant en outre l'acquisition de données concernant le flux de fluide à travers le système CFF automatisé à l'aide de la pluralité de capteurs.

10. Procédé selon l'une quelconque des revendications 8-9, dans lequel le module de filtration CFF inclut des capteurs capables de déterminer ses performances, de raccorder le réseau de traitement de données électronique au module de filtration CFF de sorte à recevoir et/ou à transmettre et/ou à traiter et/ou à enregistrer des données associées au module de filtration CFF, et déterminer les performances du module de filtration CFF à l'aide du réseau de traitement de données électronique.

11. Procédé selon l'une quelconque des revendications 8-10, dans lequel
le drain est en communication fluidique avec le conduit de perméat et le conduit en boucle de recirculation ; et
le drainage de la solution de traitement du système inclut la mise en circulation de la solution de traitement de système depuis le premier réservoir à travers le conduit d'alimentation, le module de filtration CFF automatisé, le conduit de rétentat, le conduit de perméat, le conduit en boucle de recirculation jusqu'au drain de telle sorte que l'écoulement dans le conduit de rétentat se poursuive vers le conduit en boucle de recirculation et que le flux provenant du conduit en boucle de recirculation et le flux provenant du conduit de perméat sortent du système CFF automatisé par le drain.

12. Procédé selon l'une quelconque des revendications 8-11, dans lequel la solution de traitement de système inclut un tampon et/ou dans lequel la solution pour une filtration inclut un biofluide.

13. Procédé selon l'une quelconque des revendications 8-12, dans lequel le réseau de traitement de données électronique commande les étapes consistant à :
configurer la pluralité de pompes et la pluralité de vannes pour fournir une solution de traitement de système au premier réservoir à partir du deuxième réservoir ;
fournir la solution de traitement de système au premier réservoir à partir du deuxième réservoir ;
configurer la pluralité de pompes et la pluralité de vannes pour faire circuler la solution de traitement de système depuis le premier réservoir à travers le conduit d'alimentation, le module de filtration CFF/TFF, le conduit de rétentat, le conduit de perméat et le conduit en boucle de recirculation pour retourner au premier réservoir ;
faire circuler la solution de traitement de système depuis le premier réservoir à travers le conduit d'alimentation, le module de filtration CFF, le conduit de rétentat, le conduit de perméat et le conduit en boucle de recirculation pour retourner au premier réservoir ;
configurer la pluralité de pompes et la pluralité de vannes pour drainer la solution de traitement de système à partir du système CFF automatisé à l'aide du drain ;
drainer la solution de traitement de système à partir du système CFF automatisé à l'aide du drain ;
configurer la pluralité de pompes et la pluralité de vannes pour fournir une solution pour filtration au premier réservoir à partir du troisième réservoir ;
fournir la solution pour une filtration au premier réservoir à partir du troisième réservoir ;
configurer la pluralité de pompes et la pluralité de vannes pour filtrer la solution pour une filtration depuis le premier réservoir à travers le module de filtration CFF, et
filtrer la solution pour une filtration depuis le premier réservoir à travers le module de filtration CFF,
dans lequel l'ensemble de circuits de fluide reste inchangé pendant les étapes ci-dessus.

14. Procédé selon l'une quelconque des revendications 8-13, dans lequel au moins une pompe de la pluralité de pompes est une pompe à piston et la pompe à piston, la tubulure flexible, la pluralité de capteurs, le premier réservoir, le deuxième réservoir, le troisième réservoir et le module de filtration CFF sont à usage unique.
